# EUROPEAN PATENT SPECIFICATION

(11) **EP 2 585 152 B1**
(45) Date of publication and mention of the grant of the patent: **31.12.2014**
(21) Application number: 11726778.1
(22) Date of filing: 20.06.2011
(51) Int. Cl.: A61M 16/06, A61M 16/00, A61B 5/087

(54) **VENTILATION AID, VENTILATOR, SYSTEM AND METHOD FOR THE NON-INVASIVE VENTILATION OF PREMATURE INFANTS**
VENTILATION AID, VENTILATOR, SYSTEM AND METHOD FOR THE NON-INVASIVE VENTILATION OF PREMATURE INFANTS
SYSTÈME D'ASSISTANCE DE VENTILATION, INSUFFLATEUR, SYSTÈME ET PROCÉDÉ POUR LA VENTILATION NON INVASIVE D'ENFANTS PRÉMATURÉS

(30) Priority: 22.06.2010 DE 102010030324
(43) Date of publication of application: 01.05.2013
(73) Proprietor: Heinen & Löwenstein GmbH & Co. KG, 56130 Bad Ems (DE)
(72) Inventor: BILO, Ansgar, 56132 Nievern (DE); SUSDORF, Heribert, 56566 Neuwied (DE)
(74) Representative: Vorberg, Jens
(86) International application number: PCT/EP2011/060247
(87) International publication number: WO 2011/161060

(56) References cited:
- EP-A1- 0 314 325
- EP-A1- 2 106 819
- WO-A1-97/49997
- DE-B3-102006 030 520
- US-A1- 2004 040 386
- US-A1- 2004 065 330
- MAGDALENA ZIMOVÁ-HERKNEROVÁ ET AL: "Expired tidal volumes measured by hot-wire anemometer during high-frequency oscillation in preterm infants", PEDIATRIC PULMONOLOGY, vol. 41, no. 5, 1 May 2006 (2006-05-01), pages 428-433, XP55006421, ISSN: 8755-6863, DOI: 10.1002/ppul.20367

## Description

The invention relates to a ventilation aid for the non-invasive ventilation of premature infants according to the generic portion of the patent claim 1, as well as to a ventilator, a system and a method for the non-invasive ventilation of premature infants.

Breathing is a serious problem in premature infants. The respiratory organs and thus the pulmonary function of the children are still very underdeveloped, so that a so-called idiopathic respiratory distress syndrome can occur in many cases. In the case of prolonged respiratory arrest, the brain is not supplied with oxygen to a sufficient extent. Among other things, seizures and cerebral hemorrhage may occur which cause permanent damage or even a subsequent disability.

Some premature infants, in particular very underdeveloped premature infants, require additional oxygen. Primarily the inhaled air of a child can be enriched with oxygen, for example in an incubator. However, if insufficient oxygen levels nevertheless occur in the blood of the premature infant, appropriate breathing aids have to be used.

So-called CPAP methods are being used for the ventilation of premature infants with an inadequate capability for sufficient autonomous breathing. CPAP ventilation (Continuous Positive Airway Pressure) is a form of intensive-care ventilation in which the autonomous breathing of the patient is supported by a machine, by a defined constant overpressure being continuously maintained in the supplied breathing air during the entire breathing cycle of the patient. This is supposed to prevent a collapse of the alveoli at the end of the exhalation of the patient, which is particularly relevant in the case of premature infants. CPAP therefore does not tell the patient how to breathe, but recognizes the (too-weak) autonomous breathing of the patient and boosts it in order to ensure a sufficient respiratory volume. This procedure is referred to as demand-flow. Therefore, the patient is able to determine his depth of breathing, breathing frequency and also the air flow himself. Thus, the application of a CPAP ventilation requires the capability in principle of the patient to breathe autonomously.

In the application of the CPAP ventilation, the patient to be ventilated is connected via a hose system to a ventilator machine. In the hose system, and later in the respiratory passages and the alveoli of the patient, a pressure is generated which constantly lies above atmospheric pressure. This overpressure facilitates inhalation. Apart from that, however, it makes exhalation more difficult because due to the difference to the normal pressure level, the higher ambient pressure constitutes a resistance. This higher pressure is continuously present in the ventilation system, and constantly lies a few millibars, typically, for example, 5 millibars, above the pressure of the breathing air inhaled or exhaled by the patient.

CPAP ventilation is possible both as an invasive ventilation method, that is, via a tube or tracheal cannula, as well as as a non-invasive ventilation, that is, via a mask (for example, a mouth and nose mask, nose mask, face mask or helmet).

In neonatology in particular, non-invasive CPAP ventilation of a premature infant is preferred over the invasive CPAP ventilation. In the case of NIVCPAP (non-invasive ventilation), breathing air is made available to the patient through a mask which, depending on its design, covers the area of the nose and mouth, the entire face, or was built as a full helmet. Each of these masks has only a relative tightness. The use of nasal masks or tubes is particularly preferred in the case of premature infants; this is referred to as nasal CPAP, nCPAP in short. As a rule, the masks are fixed to the patients head by means of retaining straps.

In CPAP ventilation, a mixture of air and oxygen is introduced into the lung, which prevents the lung from folding up again as the patient exhales. In this case, the patient breathes on his own. However, the autonomous breathing of the patient may fluctuate or stop, for example following the administration of tranquilizers or analgesics. In that case, CPAP ventilation cannot be used; rather, a machine takes over the breathing work of the patient entirely or partially. Advantageously, the oxygen levels in the blood of the patient are continuously monitored in the process, and only as much oxygen as is necessary is respectively administered.

In principle, it is often desirable in the case of artificial ventilation to synchronize the machine-generated ventilation cycle with the spontaneous or autonomous breathing of the patient and, if necessary, to trigger changed ventilation cycles depending on a detected spontaneous or autonomous breathing activity of the patient. This particularly applies when working with positive pressure ventilation. In the context of the present invention, this is supposed to mean an artificial ventilation of the patient in which it is ensured, by suitable measures, that there always prevails in the lung of the patient a defined overpressure ("positive pressure") relative to the atmospheric pressure. If the ventilation cycle is not synchronized with the spontaneous breathing of the patient, then this is a CPAP ventilation.

In NIV positive pressure ventilation, in particular using nasal masks or tubes, the lack of complete tightness of the aforementioned masks or tubes results in the problem of steady loss of positive pressure, and thus of breathing air, from the ventilation system, which has to be compensated by the ventilator. Furthermore, this continuous pressure loss, which, however, cannot be specified precisely, so far makes detecting the spontaneous breathing activity of the patient virtually impossible.

So far, only the use of abdominal probes, which are stuck on the patient's chest, has proved its value in practice. These probes, which are susceptible to the abdominal movements of the patient during spontaneous breathing, can be used to detect a spontaneous or autonomous breathing of the patient in order to control or regulate the positive pressure ventilation accordingly. However, abdominal probes have such significant drawbacks in the case of premature infants that their use is avoided if possible in clinical practice. For example, injuries may occur when the probes are removed, due to the very thin skin of the premature infants, which is not yet completely developed. Furthermore, unpleasant reactions of the skin, including allergic reactions, can be caused in a premature infant by the adhesive used for sticking on the abdominal probes. For this reason, the use of abdominal probes in premature infants has not found acceptance.

International Patent Application WO 2010/028150 A1 describes a ventilator with controlled purge function, for example, whereby a pressure sensor is located close to the patient. For example, a sensor may be placed at the patient wye proximal to the patient.

The present invention is based on the object of proposing a ventilation aid with which a reliable detection of the spontaneous or autonomous breathing of the premature infant can be realized without having to attach probes on the body of the premature infant.

This object is achieved by means of a system according to patent claim 1.

The dependent claims contain further advantageous features.

The invention achieves this object by configuring a generic ventilation aid in such a way that the ventilation aid comprises a pressure measuring device designed to generate a pressure signal characterizing the pressure in the area of the contact part. By means of the pressure measuring device, the pressure of the ventilation air can be measured, for example, in a mixing chamber close to the patient, or the pressure of the expired air in the area of the expiration valve, whereby the effective ventilation of the premature infant can be calculated.

For example, the pressure measuring device can be configured as a measuring line with a thin cross section separate from the ventilator into which a manometer is integrated. Alternatively, the pressure measuring device can be disposed directly in the mixing chamber or in the area of the mixing chamber of the ventilation aid, or in the ventilator itself, and be connected with the mixing chamber via a measuring hose with a small internal diameter that is additionally connected to the mixing chamber. If a certain pressure or pressure curve is detected in the ventilation aid, this supplies another clue with regard to a spontaneous or autonomous breathing activity of the premature infant (inhalation -> pressure drop, exhalation -> pressure increase). Thus, a possible spontaneous or autonomous breathing activity of the premature infant is detected not by means of probes or the like, but rather through a pressure measuring device which detects the pressure in the patient's breathing air in a suitable manner.

Moreover, the possibly detrimental influence of an airway pressure that is too high can be prevented by means of the pressure measuring device, due to the possibility of limiting pressure or fixing an upper pressure limit. It can be provided, for example, that, upon reaching a predetermined pressure value, the ventilator switches off the breathing air supply or that the overpressure in the supplied breathing air is reduced. Moreover, it is possible, in accordance with a detected low-pressure value that is below a predetermined threshold, to switch on the ventilator or to increase the provided overpressure.

In an advantageous embodiment, the pressure measurement is combined with a flow measurement in the breathing air flow in order to increase the accuracy of the entire system. To this end, at least one sensor is disposed in the ventilation air flow which is configured to detect a measuring parameter characterizing the ventilation air flow and to generate a corresponding flow signal.

For example, the pressure measuring device and/or the sensor can be used in closed or open hose systems (leakage, single or double hose systems). The hose systems can comprise an active expiration valve controlled by the ventilator. As a rule, it is located distant from the patient, i.e. in or on the ventilator, optionally also in the ventilation hose. When the premature infant breathes in, the expiration valve is in an inspiration position in which an overpressure present in the hose reaches the mask and thus the lung of the premature infant. An expiration aperture is closed so that the desired overpressure of the ventilation air is able to build up in the mask. During exhalation, the expiration valve is in an expiration position in which the exhaled air can escape through the expiration aperture with as little resistance as possible. The supply of the fresh ventilation air as well as the leading off of the used-up ventilation air defines the ventilation air flow in which the at least one sensor is disposed.

Preferably, the sensor is configured to detect at least the strength and/or the flow direction of the ventilation air flow. The sensor permits conclusions to be drawn with regard to a spontaneous or autonomous breathing activity of the premature infant setting in or being present. It is configured to generate a corresponding flow signal.

One embodiment provides that the sensor is disposed on the ventilation aid or integrated therein. For example, the sensor can be disposed in a separate chamber or recess, through which the ventilation air flow flows, on or in the ventilation aid, or protrude into the ventilation air flow from such a chamber. Such a chamber can comprise connections for a hose for breathing air supply, a hose for breathing air discharge, and a port for connecting a face mask (CPAP), a nasal mask or a nasal tube (both nCPAP).

Relative to a flow-through direction of the ventilation air flow, the sensor is disposed between the contact part of the ventilation aid and the mixing chamber. It is advisable to dispose the sensor(s) as closely as possible to the area of the contact part or the ventilation aid in order to be able to detect the spontaneous or autonomous breathing activity of the premature infant with as little a delay in time as possible.

Preferably, the sensor can be configured as a hot-wire anemometer. In hot-wire anemometry, a very thin wire is generally used which typically has a diameter of 2.5 - 10 µm. The materials used include platinum, nickel, tungsten and other different alloys, depending on the requirements with regard to its physical properties. The measuring principle is based on an electrically heated wire whose thermal output to the air flowing past serves for determining the flow velocity.

For example, the hot-wire anemometer can be configured as a double-wire anemometer. The double-wire version determines the flow velocity through the temperature difference between the wires, one of which lies in the air flow and the other in the lee. The advantage of the double-wire anemometer is that the flow direction can be determined from the sequence of which of the wires cools off first.

A preferred embodiment provides that the ventilation aid is configured as a nasal mask. For example, the nasal mask can consist of silicone. The nasal mask comprises a contact part which comes into contact with the area of the nose and mouth of the premature infant. Straps or belts provide for fixation and ensure that the nasal mask is placed securely on the head of the premature infant.

Another preferred embodiment provides that the ventilation aid is configured as a nasal tube or nasal prong. For example, the nasal tube is configured as a double tube with two hose ends from a flexible material that can introduced through the nostrils, and a rigid connecting element, which is connected thereto, for the hose system or the ventilator.

A ventilator with a controllable breathing gas source, in particular with a pressure container, which is filled with a pressurized breathing gas and which, via regulating and shut-off devices, is suitable for outputting a breathing gas to a ventilation aid. The ventilator comprises a control device configured to detect and process a pressure signal characterizing the pressure or pressure curve in the area of the contact part of the ventilation aid, e.g. in a mixing chamber on the side of a patient, the ventilator being configured to generate a ventilation cycle predefined by the control device. Thus, the ventilator comprises a controller for the entire ventilation cycle, taking into account at least the sensor results. The ventilation cycle (or breathing cycle) defines the period of time between the start of two successive strokes of breath. Each breathing cycle consists of an inspiration phase and an expiration phase. The ventilation frequency describes the frequency in which the individual cycles comprised of inspiration and expiration recur. Within the ventilation cycle, there are certain pressure and flow conditions that vary over time ("pressure profile", "flow profile") in the lung of the patient, and thus in the ventilation aid according to the invention. If a ventilation cycle is predetermined automatically, at least the parameters ventilation frequency, pressure profile and flow profile can thus be selected set individually or in combination.

For ventilation purposes, a continuous air flow with a slight overpressure is as a rule supplied from the control device via a feed line to the ventilation aid. The air flow is returned via a returning line from the ventilation aid to the ventilator.

For monitoring purposes, the ventilator can, for example, comprise means for the numerical and/or graphic display of pressure, flow and/or volume, or a display of the average values of all ventilation parameters.

The combination of a suitably configured ventilator with a ventilation aid according to the invention makes it possible to synchronize the automatically generated ventilation cycle with the spontaneous or autonomous breathing activity of the premature infant. Moreover, a change of the ventilation cycle applied by the machine can be effected depending on the detected spontaneous or autonomous breathing activity of the patient. To this end, the control device can, for example, be configured to control the ventilation cycle depending on the pressure signal characterizing the pressure or pressure curve in the area of the contact part of the ventilation aid, for example in a mixing chamber on the side of the patient. Also in this case, the control device can apply or effect a different control algorithm and thus a different ventilation cycle if the pressure signal of the pressure measuring device suggests a (changed) spontaneous or autonomous breathing activity of the premature infant.

Particular advantages are the result if the pressure signal as a whole, preferably a variable part of the pressure signal, which is supplied to the control device, undergoes a powerful amplification prior to processing in the control device, in particular is amplified at least by the factor 5, preferably by the factor 10, particularly preferably by the factor 50. According to the invention, the variable part is obtained from determining a difference between the detected actual pressure value and a corrective value K, wherein the corrective value can be correlated with the current status of the breathing cycle of the patient detected by the control device. In particular, the corrective value K can correlate directly to the aperture value of the expiration valve, e.g. in a proportional manner.

Apart from the amplification of the pressure signal, the application of algorithms for pattern recognition in the control device has proved advantageous. This method is based on the knowledge that the behavior over time both of the pressure signal as well as of a flow signal - which will be defined in more detail later - always follows a general pattern despite all practical variabilities and interferences. The digitization possible today of the measuring parameters detected over time that characterize the autonomous breathing of the patient permits the application of algorithms in the control unit in a simple manner, with the algorithms being based, for example, on the numerical determination of correlation functions. The pattern recognition proposed according to the invention is used, in particular, to recognize and "gate" artifacts in the detected measuring parameters.

According to the invention, the control device is further configured to detect and evaluate the flow signal of a sensor disposed in the ventilation air flow in order to control the ventilation cycle also depending on the flow signal of the sensor. This means that the control device applies or effects a different control algorithm and thus a different ventilation cycle if the pressure signal of the sensor in the ventilation aid suggests a (changed) spontaneous or autonomous breathing activity of the premature infant.

Moreover, the ventilator can be configured to control the composition of the breathing gas discharged by the breathing gas source to the ventilation aid. A gas mixture used for breathing activities with pressurized-air breathing apparatus is generally referred to as a breathing gas. Every gas mixture provided for breathing has to contain oxygen. Air (pressurized air), for example, is the most widespread breathing gas mixture and, simply speaking, consists of 79% nitrogen and 21 oxygen as well as residues of carbon dioxide and noble gasses. The ventilator can be configured to control or change these values depending on the application or the individual adjustment to a premature infant.

The system according to the invention can be used for a method for the non-invasive ventilation of premature infants, wherein the method can comprise at least the following method steps:
a. supplying a controllable ventilation gas flow to the premature infant;
b1. detecting and analyzing a pressure signal which characterizes the pressure curve in the ventilation gas flow with regard to its interaction with the premature infant,
c. generating a control signal at least from the pressure signal, the control signal characterizing the spontaneous breathing of the premature infant; and
d. controlling the flow curve in the ventilation gas flow by means of the control signal.

The method can further be characterized by the following additional method step: b2. detecting a flow signal characterizing the change of the ventilation gas flow caused by the spontaneous breathing of the premature infant, with the control signal being generated at least from the pressure signal and the flow signal in step c.

It is thus possible, with the help of the invention, to synchronize an automatically generated ventilation cycle with the spontaneous or autonomous breathing of a premature infant and to effect, depending on a detected (changed) spontaneous or autonomous breathing activity of the premature infant, changed ventilation cycles. Express reference is made to the fact that it lies within the scope of the present invention to exchange for one another the roles of the two detected parameters characterizing the spontaneous breathing of the patient, i.e. the flow signal and the pressure signal. Thus, the positive pressure ventilation would primarily be synchronized to the flow signal, and the pressure signal would be taken into account only secondarily as a corrective parameter. This exchange in this case pertains to all developments of the invention described herein, i.e. to the system according to claim 1.

Other advantages, features and details of the invention become apparent from the following description, in which an exemplary embodiment of the invention is described with reference to the drawing. In this case, the features mentioned in the claims and in the description, both individually and in any combination, can be fundamental to the invention.

In the drawings:

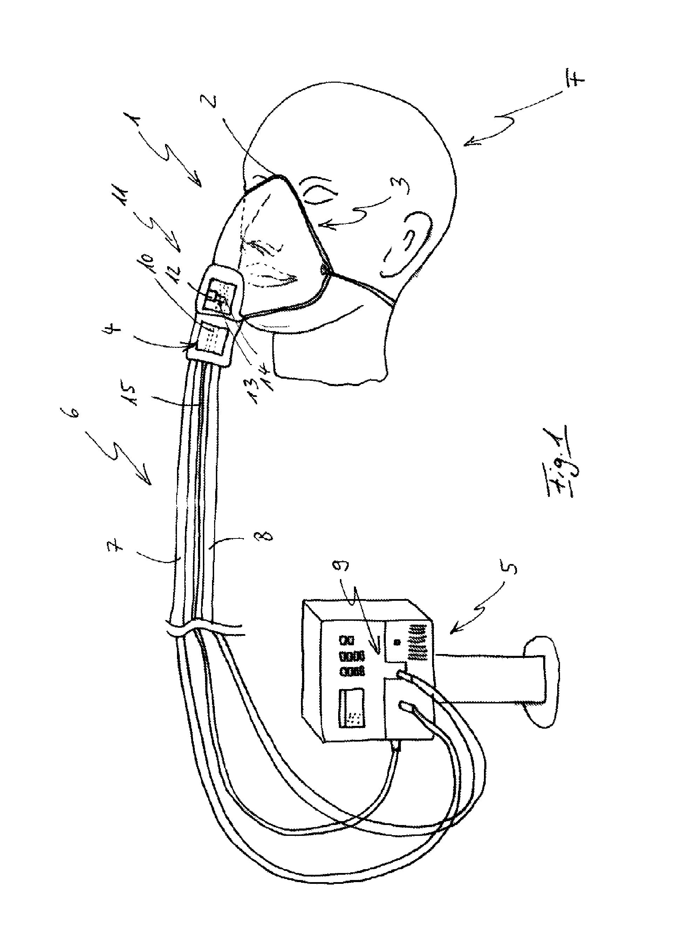

Fig. 1 shows a schematic representation of a system according to the invention for the non-invasive ventilation of premature infants by means of positive pressure ventilation.

Fig. 1 shows a schematic representation of a system for the non-invasive ventilation of a premature infant F. The system comprises a ventilation aid 1 which is configured as a nasal mask and has a contact part 2. With this contact part 2, the ventilation aid 1 comes into contact with the nose/mouth area 3 of the premature infant F.

The system further comprises a controllable ventilator 5 connected to a hose system 6 belonging to the ventilation aid 1. The hose system 6 comprises a feed line 7 for supplying fresh ventilation air to the premature infant F and a returning line 8 for leading off used-up ventilation air from the premature infant F. The ventilator 5 is configured to generate a ventilation cycle predetermined by a control device provided in the ventilator 5.

The ventilator 5 is configured to generate a constant ventilation air flow with a controlled overpressure fed into the feed line 7.

As was mentioned, the ventilation aid 1 is formed like a nasal mask and comprises a mixing chamber 4 comprising an inlet for the feed line 7 of fresh ventilation air and an outlet for the returning line 8 of the used-up ventilation air to the ventilator 5. The feed line 7 and the returning line 8 are connected through these connections with the mixing chamber 4 of the ventilation aid 1.

An expiration valve 9 which is connected to the device end of the returning line 8 and controls the airflow therein is disposed in the ventilator 5. To this end, the expiration valve 9 is controlled by the control device. When the premature infant breathes in, the expiration valve 9 is in an inspiration position in which an overpressure present in the feed line 7 reaches the mask and thus the lung of the premature infant. An expiration aperture is closed so that the desired overpressure of the ventilation air is able to build up in the mask. During exhalation, the expiration valve 9 is in an expiration position in which the exhaled air can escape through the expiration aperture with as little resistance as possible. The supply of the fresh ventilation air as well as the leading off of the used-up ventilation air defines the ventilation air flow, which is controlled by the ventilator 5 and in which the at least one sensor is disposed.

The ventilation aid 1 moreover comprises a sensor chamber 11 in which a hot-wire anemometer 12 is disposed. The hot-wire anemometer 12 is configured like a double-wire anemometer. The hot-wire anemometer 12 is arranged in the sensor chamber 11 in such a way that the two hot wires 13 and 14 are disposed in the ventilation air flow 10, or that it flows around them. Relative to the flow-through direction of the ventilation air flow 10, the sensor 12 is disposed between the contact part 2 and the mixing chamber 4.

The hot-wire anemometer 12 is configured to detect a measuring parameter characterizing the ventilation air flow 10, in particular the strength and the flow direction of the ventilation air flow 10 and to generate a corresponding control signal. The control signal is forwarded through a connection line 15 to the control device of the ventilator 5.

Furthermore, a pressure measuring line 16 connected to a pressure measuring device 17 disposed in the ventilator 5 is disposed on the mixing chamber 4. In this case, the internal diameter of the pressure measuring line 16 is selected such that the pressure curve at the device end of the pressure measuring line 16 substantially follows the pressure curve in the mixing chamber 4 without any substantial delay in time (i.e. ≤ 0.5 sec, preferably ≤ 0.2 sec). Alternatively, a suitable pressure measuring device 17 can be disposed close to the patient, on the ventilation aid 1, in particular the mixing chamber 4, in order to generate a pressure signal. This embodiment results in the advantage of an even smaller delay in time between the flow signal of the anemometer and the pressure signal of the pressure measuring device 17, whereby the quality of correlation of the two signals can be improved even further.

The pressure measuring device 17 generates a pressure signal which is also fed to the control device and amplified there. The control device analyzes the pressure signal using suitable algorithms for pattern recognition in order to recognize a spontaneous or autonomous breathing activity of the premature infant. If there is a spontaneous or autonomous breathing activity of the premature infant F, then the pressure signal is used in such a way that the ventilation cycle generated automatically by the ventilator 5 is changed and synchronized with the spontaneous or autonomous breathing activity of the premature infant F.

Optionally, the pressure measurement signal is additionally correlated in the control device with the control signal of the anemometer 12, with the correlation being taken into account when the breathing cycle is determined by the ventilator 5. It is possible, by means of a suitable correlation of the flow signal from the anemometer 12 with the pressure signal, to recognize the breathing cycle of the newborn child with an even greater reliability, despite the constant losses of breathing gas from the ventilation system, which cannot be predicted with certainty, and to synchronize the ventilation cycle generated by the ventilator 5 with the spontaneous breathing cycle of the newborn child F.

**Reference numerals**

| | | | |
|---|---|---|---|
| 1 | Ventilation aid | 10 | Ventilation air flow |
| 2 | Contact part | 11 | Sensor chamber |
| 3 | Nose/mouth area | 12 | Anemometer |
| 4 | Mixing chamber | 13 | Hot wire |
| 5 | Ventilator | 14 | Hot wire |
| 6 | Hose system | 15 | Connection line |
| 7 | Feed line | 16 | Pressure measuring line |
| 8 | Returning line | 17 | Pressure measuring device |
| 9 | Expiration valve | | |

## Claims

1. System for the non-invasive ventilation of premature infants (F), comprising a ventilation aid (1) and a ventilator,
wherein the ventilation aid (1) comes into contact with the nose/mouth area (3) of the premature infant (F) with a contact part (2), and wherein the ventilation aid (1) comprises at least one mixing chamber (4) and a ventilation hose system (6) that can be connected to a controllable ventilator (5), wherein the ventilation hose system (6) comprises at least one feed line (7) for supplying fresh ventilation air to the premature infant (F) and at least one returning line (8) for leading off used-up ventilation air from the premature infant (F), and wherein the ventilation aid (1) comprises a pressure measuring device (17) configured to generate a pressure signal characterizing the pressure in the area of the contact part (2), whereby spontaneous breathing of the premature infant and the supplying and/or leading off of the ventilation air within the ventilation aid (1) defines a ventilation air flow (10), wherein at least one sensor (12) is disposed in the ventilation air flow (10) which is configured to detect a measuring parameter characterizing the ventilation air flow (10) and to generate a corresponding flow signal, and that the sensor (12) is disposed between the contact part (2) and the mixing chamber (4), relative to a flow direction of the ventilation air flow (10), and the ventilator (5) comprises a controllable breathing gas source for outputting a breathing gas to the ventilation aid (1), wherein the ventilator (5) comprises a control device configured to generate, dependent on a pressure signal characterizing the pressure in the area of the contact part (2) of the ventilation aid (1), a predefined ventilation cycle, and that the control device is further configured to detect and evaluate the flow signal of the sensor (12) disposed in the ventilation air flow (10), and the ventilator (5) is further configured to control the ventilation cycle depending on the flow signal of the sensor (12), whereby a variable part of the pressure signal is amplified prior to processing in the control device, wherein the variable part is obtained from determining a difference between the detected actual pressure value and a corrective value K and the corrective value K is correlated with the current status of the breathing cycle of the patient detected by the control device.

2. System according to claim 1, **characterized in that** the ventilation aid (1) is configured as a nasal mask.

3. System according to claim 1, **characterized in that** the ventilation aid is configured as a nasal tube.

4. System according to any one of claims 1-3, **characterized in that** the sensor (12) is configured to detect at least the strength and/or the flow direction of the ventilation air flow (10).

5. System according to any one of claims 1-3, **characterized in that** the sensor (12) is disposed on or integrated into the ventilation aid (1).

6. System according to any one of claims 1-3, **characterized in that** the sensor (12) is configured as a hot-wire anemometer.

7. System according to claim 6, **characterized in that** the hot-wire anemometer (12) is configured as a double-wire anemometer.

8. System according to any one of claims 1-7, **characterized in that** the ventilator (5) is configured to control the composition of the breathing gas discharged by the breathing gas source to the ventilation aid (1).

## Patentansprüche

1. System für die nicht-invasive Beatmung Frühgeborener (F), aufweisend eine Beatmungshilfe (1) und einen Ventilator, wobei die Beatmungshilfe (1) mit einem Kontaktteil (2) in Berührung mit dem Nasen-/Mundbereich (3) des Frühgeborenen (F) kommt und wobei die Beatmungshilfe (1) wenigstens eine Mischkammer (4) und ein Beatmungsschlauchsystem (6), das mit einem regulierbaren Ventilator (5) verbunden werden kann, aufweist, wobei das Beatmungsschlauchsystem (6) wenigstens eine Zuführleitung (7), um dem Frühgeborenen (F) frische Beatmungsluft zuzuführen, und wenigstens eine Rückführleitung (8) zum Ableiten verbrauchter Beatmungsluft von dem Frühgeborenen (F) aufweist, und wobei die Beatmungshilfe (1) eine Druckmessvorrichtung (17) aufweist, die ausgelegt ist, ein Drucksignal zu erzeugen, das den Druck in dem Bereich des Kontaktteils (2) kennzeichnet, wobei ein spontanes Atmen des Frühgeborenen und das Zuführen und/oder Ableiten der Beatmungsluft innerhalb der Beatmungshilfe (1) einen Beatmungsluftstrom (10) definieren, wobei wenigstens ein Sensor (12) in dem Beatmungsluftstrom (10) angeordnet ist, der ausgelegt ist, einen Messparameter zu detektieren, der den Beamtungsluftstrom (10) kennzeichnet, und ein entsprechendes Strömungssignal zu erzeugen, und dass der Sensor (12) zwischen dem Kontaktteil (2) und der Mischkammer (4) bezüglich einer Strömungsrichtung des Beatmungsluftstroms (10) angeordnet ist und der Ventilator (5) eine regulierbare Atemgasquelle zur Förderung eines Atemgases zu der Beatmungshilfe (1) aufweist, wobei der Ventilator (5) eine Steuereinrichtung aufweist, die ausgelegt ist, abhängig von einem Drucksignal, das den Druck in dem Bereich des Kontaktteils (2) der Beatmungshilfe (1) kennzeichnet, einen vorbestimmten Beatmungszyklus zu erzeugen, und dass die Steuereinrichtung ferner ausgelegt ist, das Strömungssignal des Sensors (12), der in dem Beatmungsluftstrom (10) angeordnet ist, zu detektieren und auszuwerten, und der Ventilator (5) ferner ausgelegt ist, den Beatmungszyklus abhängig von dem Strömungssignal des Sensors (12) zu steuern, wobei ein variabler Teil des Drucksignals vor der Verarbeitung in der Steuereinrichtung verstärkt wird, wobei der variable Teil aus der Ermittlung einer Differenz zwischen dem detektierten tatsächlichen Druckwert und einem Korrekturwert K erhalten wird und der Korrekturwert K mit dem Momentanzustand des Atemzyklus des Patienten, der durch die Steuereinrichtung detektiert wird, korreliert ist.

2. System nach Anspruch 1, **dadurch gekennzeichnet, dass** die Beatmungshilfe (1) als eine Nasalmaske ausgelegt ist.

3. System nach Anspruch 1, **dadurch gekennzeichnet, dass** die Beatmungshilfe (1) als ein Nasalschlauch ausgelegt ist.

4. System nach einem der Ansprüche 1-3, **dadurch gekennzeichnet, dass** der Sensor (12) ausgelegt ist, wenigstens die Stärke und/oder die Strömungsrichtung des Beatmungsluftstroms (10) zu detektieren.

5. System nach einem der Ansprüche 1-3, **dadurch gekennzeichnet, dass** der Sensor (12) auf der Beatmungshilfe (1) angeordnet oder in diese integriert ist.

6. System nach einem der Ansprüche 1-3, **dadurch gekennzeichnet, dass** der Sensor (12) als ein Hitzdrahtanemometer ausgelegt ist.

7. System nach Anspruch 6, **dadurch gekennzeichnet, dass** das Hitzdrahtanemometer (12) als ein Doppeldrahtanemometer ausgelegt ist.

8. System nach einem der Ansprüche 1-7, **dadurch gekennzeichnet, dass** der Ventilator (5) ausgelegt ist, die Zusammensetzung des Atemgases zu steuern, das von der Atemgasquelle an die Beatmungshilfe (1) abgegeben wird.

## Revendications

1. Système pour la ventilation non-invasive de prématurés (F), comprenant une aide à la ventilation (1) et un ventilateur, dans lequel ladite aide à la ventilation (1) entre en contact, avec une partie de contact (2), avec la zone de nez/bouche (3) du prématuré (F) et dans lequel ladite aide à la ventilation (1) comprend au moins une chambre de mélange (4) et un système de tuyau flexible de ventilation (6) qui peut être connecté à un ventilateur (5) réglable, dans lequel ledit système de tuyau flexible de ventilation (6) comprend au moins une conduite d'alimentation (7) pour amener de l'air frais de ventilation au prématuré (F) ainsi qu'au moins une conduite de retour (8) pour évacuer de l'air vicié de ventilation depuis le prématuré (F), et dans lequel ladite aide à la ventilation (1) présente un dispositif de mesure de pression (17) configuré pour générer un signal de pression caractérisant la pression dans la zone de la partie de contact (2), une respiration spontanée du prématuré et l'amenée et/ou l'évacuation de l'air de ventilation à l'intérieur de ladite aide à la ventilation (1) définissant un courant d'air de ventilation (10), dans lequel au moins un capteur (12) est disposé dans le courant d'air de ventilation (10), qui est configuré pour détecter un paramètre de mesure caractérisant le courant d'air de ventilation (10), et pour générer un signal d'écoulement correspondant, et que ledit capteur (12) est disposé entre ladite partie de contact (2) et ladite chambre de mélange (4) par rapport à une direction d'écoulement du courant d'air de ventilation (10), et ledit ventilateur (5) comprend une source réglable de gaz respiratoire destinée à fournir un gaz respiratoire à ladite aide à la ventilation (1), dans lequel le ventilateur (5) comprend un dispositif de commande configuré pour générer, en fonction d'un signal de pression caractérisant la pression dans la zone de la partie de contact (2) de l'aide à la ventilation (1), un cycle de ventilation prédéfini, et que le dispositif de commande est configuré en outre pour détecter et évaluer le signal d'écoulement du capteur (12) disposé dans le courant d'air de ventilation (10), et ledit ventilateur (5) est configuré en outre pour commander le cycle de ventilation en fonction du signal d'écoulement du capteur (12), une partie variable du signal de pression étant amplifiée avant le traitement dans ledit dispositif de commande, dans lequel la partie variable est obtenue en déterminant une différence entre la valeur de pression réelle détectée et une valeur de correction K et ladite valeur de correction K est corrélée avec l'état momentané du cycle de respiration du patient détecté par ledit dispositif de commande.

2. Système selon la revendication 1, **caractérisé par le fait que** ladite aide à la ventilation (1) est configurée comme un masque nasal.

3. Système selon la revendication 1, **caractérisé par le fait que** ladite aide à la ventilation (1) est configurée comme un tuyau flexible nasal.

4. Système selon l'une quelconque des revendications 1 à 3, **caractérisé par le fait que** ledit capteur (12) est configuré pour détecter au moins l'intensité et/ou la direction d'écoulement du courant d'air de ventilation (10).

5. Système selon l'une quelconque des revendications 1 à 3, **caractérisé par le fait que** ledit capteur (12) est disposé sur ou est intégré à ladite aide à la ventilation (1).

6. Système selon l'une quelconque des revendications 1 à 3, **caractérisé par le fait que** ledit capteur (12) est configuré comme un anémomètre à fil chaud.

7. Système selon la revendication 6, **caractérisé par le fait que** ledit anémomètre à fil chaud (12) est configuré comme un anémomètre à fil double.

8. Système selon l'une quelconque des revendications 1 à 7, **caractérisé par le fait que** ledit ventilateur (5) est configuré pour commander la composition du gaz respiratoire distribué par la source de gaz respiratoire à ladite aide à la ventilation (1).
